# EUROPÄISCHE PATENTSCHRIFT

(11) **EP 2 788 209 B1**
(45) Veröffentlichungstag und Bekanntmachung des Hinweises auf die Patenterteilung: **06.01.2016**
(21) Anmeldenummer: 12794304.1
(22) Anmeldetag: 27.11.2012
(51) Int. Cl.: B60G 21/055

(54) **ACHSE EINES ZWEISPURIGEN FAHRZEUGS MIT EINEM DREHSTABSTABILISATOR**
AXLE FOR A TWO-TRACK VEHICLE, COMPRISING A TORSION BAR STABILIZER
ESSIEU DE VÉHICULE À DEUX VOIES COMPRENANT UN STABILISATEUR À BARRE DE TORSION

(30) Priorität: 09.12.2011 DE 102011088179
(43) Veröffentlichungstag der Anmeldung: 15.10.2014
(73) Patentinhaber: Bayerische Motoren Werke Aktiengesellschaft, 80809 München (DE)
(72) Erfinder: DURKOVIC, Martin, 80809 München (DE)
(86) Internationale Anmeldenummer: PCT/EP2012/073754
(87) Internationale Veröffentlichungsnummer: WO 2013/083441

(56) Entgegenhaltungen:
- EP-A2- 1 564 041
- DE-A1- 4 411 285
- DE-A1-102004 020 073
- DE-A1-102006 054 874
- JP-A- H09 164 830
- US-A- 4 632 422
- US-B1- 6 250 660

## Beschreibung

Die Erfindung betrifft eine Achse für Räder eines zweispurigen Fahrzeugs, insbesondere eines zweispurigen Kraftfahrzeugs, mit einer einen Radträger führenden, aufgelösten unteren Lenkerebene, die durch einen Querlenker sowie einen Längslenker gebildet ist, sowie mit einem Drehstabstabilisator, der um eine im wesentlichen in Fahrzeug-Querrichtung verlaufende Drehachse verdrehbar und mit seinem Endbereich über eine drehmomentübertragende Verbindung mit dem vom Radträger abgewandten Endbereich des Längslenkers verbunden ist.

Derartige Achsen werden als so genannte Zweigelenk-Federbeinachse oder Doppelquerlenkerachse ausgeführt und insbesondere bei Kraftfahrzeugen in Gestalt von Personenkraftwagen verbaut. Die Achse bildet in der Regel die Lenkachse mit lenkbaren Rädern bzw. Vorderachse des jeweiligen Fahrzeugs. Die Achse ist an ihrer unteren Lenkerebene mit einem sich im Wesentlichen quer zur Hauptfahrtrichtung erstreckenden Querlenker zum Aufnehmen und Überleiten hauptsächlich der Querkräfte der Räder an die Karosserie des Fahrzeugs gestaltet. Ferner ist an der unteren Lenkerebene ein Längslenker, auch als Zugstrebe bezeichnet, vorgesehen, der sich in der Regel schräg zur Hauptfahrtrichtung erstreckt und dazu dient im Wesentlichen die Radlängskräfte aufzunehmen.

An solchen Achsen ist ferner ein Drehstabstabilisator vorgesehen, der die beiden Spurseiten der Achse miteinander verbindet und zum Reduzieren der Wankneigung des Fahrzeugs dient. Der Drehstabstabilisator ist dazu in der Regel zumeist am Radträger abgestützt. Bei bekannten Zweigelenk-Federbeinachsen kann der Drehstabstabilisator auch an dessen Federbein abgestützt sein. Das Abstützen erfolgt dabei üblicherweise über eine Pendelstütze, welche mit zwei Kugelgelenken oder Gummigelenken an einem Ende an dem dabei an seinen Endabschnitten abgebogenen Drehstabstabilisator befestigt ist. Der Drehstabstabilisator ist ferner in der Regel mit seinem Querstab selbst an zwei Halterungen bzw. Abstützungen direkt oder indirekt am Fahrzeugaufbau abgestützt.

Aus DE 10 2004 020 073 A1 ist eine Zweigelenk-Federbeinachse oder Doppelquerlenkerachse bekannt, bei der das dem Radträger abgewandte Ende des im wesentlichen Rad-Längskräfte aufnehmenden Längslenkers über ein Drehgelenk, dessen Drehachse winkelig zur Drehachse des Drehstabstabilisators verläuft, mit dem zugewandten freien Ende des Drehstabstabilisators verbunden ist. Damit kann eine im Stand der Technik übliche Pendelstütze entfallen. Nach dem gleichen Prinzip, jedoch andersartig gestaltet, ist bei der JP-H-9-164830 eine solche Verbindung mit einem Schwenkgelenk gebildet, bei dem der Endbereich des Drehstabilisators den Endbereich des Längslenkers schwenkbar umgreift. Dabei erscheint diese bekannt Konstruktion jedoch hinsichtlich der Führungsgenauigkeit verbesserungswürdig.

Letzteres ist Aufgabe der vorliegenden Erfindung, d.h. es soll eine bauraumsparende Achse für Räder eines zweispurigen Fahrzeugs geschaffen werden, die bei gutem Einfederungsverhalten hohe Anforderungen hinsichtlich einer möglichst exakten Radführung erfüllt.

Die Lösung dieser Aufgabe ergibt sich für eine Achse nach dem Oberbegriff des Anspruchs 1 mit den kennzeichnenden Merkmalen dieses Anspruchs.

Der erfindungsgemäß vorgesehene Umgriff des Endbereichs des Drehstabstabilisators um den Endbereich des Längslenkers herum ermöglicht es, dass der äußere Teil des Schwenkgelenks vom Drehstabstabilisator selbst ausgebildet werden kann. Der Drehstabstabilisator ist herkömmlich ein stabförmiges Bauteil aus einem hochwertigen Federstahl und kann daher nur begrenzt formend bearbeitet werden. Mit der erfindungsgemäßen Lösung ist es trotz der schwierigen Bearbeitbarkeit möglich, den Drehstabstabilisator auf günstige Weise drehmomentenübertragend und dennoch nicht vollständig starr an den Längslenker zu koppeln. Dabei umgreift der Endbereich des Drehstabstabilisators umgreift den Endbereich des Längslenkers im Wesentlichen zumindest halbkreisförmig, wofür der Endbereich des Längslenkers zumindest annähernd kreisringscheibenförmig ausgebildet sein kann. Mit einer Halbkreisform ist eine vergleichsweise große Kontaktfläche geschaffen, was vorteilhafterweise zu einem entsprechend geringen partiellen Flächendruck führt.

Das Schwenkgelenk ist dabei bevorzugt als ein Gleitlager des Drehstabstabilisators am Längslenker gestaltet. Der Drehstabstabilisator gleitet bei dieser Weiterbildung also um den Endbereich des Längslenkers herum und hält diesen dabei schalenförmig bzw. bogenförmig innerhalb seiner Form. Es ist auf diese Weise ein gleitender Formschluss zwischen dem Drehstabstabilisator und dem Längslenker geschaffen.

Der Längslenker ist vorzugsweise mit einem Dämpfungskörper zum federnden Abstützen des Längslenkers an einer zugehörigen Fahrzeugkarosserie gestaltet und der Dämpfungskörper ist dabei vorzugsweise im Endbereich des Längslenkers angeordnet. Mit dem Dämpfungskörper ist zum einen eine Schallentkopplung zwischen Längslenker und Fahrzeugkarosserie geschaffen und zum anderen sichergestellt, dass Schwingungen und Stöße der Radanordnung möglichst gedämpft werden.

Besonders bevorzugt ist der Dämpfungskörper im Zentrum des Schwenkgelenks angeordnet. Der Dämpfungskörper ist dann vom Endbereich des Drehstabstabilisators umfasst, wodurch insgesamt eine besonders bauraumsparende Anordnung geschaffen ist. Ferner besteht der Umgriff in gerade jenem Bereich des Längslenkers an dem dieser dämpfend an der Fahrzeugkarosserie angelenkt ist. Der Längslenker schwenkt also in jenem Bereich, in dem sich auch die Kopplung zum Drehstabstabilisator befindet. Da auch der Drehstabstabilisator bei der Drehmomentenübertragung bzw. im Rahmen seiner funktionsgemäßen Torsion eine gewisse Drehbewegung und damit Relativbewegung zum Längslenker durchführt, können mit der erfindungsgemäßen Weiterbildung vorteilhaft beide Bewegungen mit praktisch einer Gelenkanordnung kompensiert werden. Diese Gelenkanordnung umfasst vorteilhaft den genannten Dämpfungskörper, da dieser selbst nachgiebig ist und daher seinerseits Relativbewegungen spannungsarm ausgleichen kann.

Der Längslenker ist vorteilhaft mit einem Gelenk zum Abstützen des Längslenkers an einer zugehörigen Fahrzeugkarosserie gestaltet und das Gelenk ist dabei bevorzugt als Kugelgelenk gestaltet. Das Kugelgelenk lässt eine Relativbewegung in zwei Dimensionen zu und schafft damit zugleich eine Abstützung in einer dritten Dimension, mittels der eine weitgehend spannungsarme Drehmomentenübertragung zwischen den beteiligten Bauteilen Längslenker und Drehstabstabilisator möglich wird.

Das Kugelgelenk ist vorzugsweise im Zentrum des Schwenkgelenks angeordnet. Der derart mit einem Kugelgelenk an einer Fahrzeugkarosserie angelenkte Längslenker ist zentral schwenkbar gehalten und zugleich derart vom Endbereich des Drehstabstabilisators umgriffen, dass der Umgriff in der Art einer Kardankopplung ein Drehmoment überträgt. Zur dämpfenden Lagerung des Längslenkers ist es dabei ferner vorteilhaft möglich, im Inneren des Kugelgelenks einen Dämpfungskörper anzuordnen.

Ferner ist das Kugelgelenk bevorzugt mit mindestes einer seitlichen Abflachung zum Festlegen einer definierten Rotationsachse des Längslenkers an der Fahrzeugkarosserie versehen. Mit dem Kugelgelenk kann auf diese Weise ein Schwenkgelenk mit im Wesentlichen nur einem Freiheitsgrad geschaffen werden. Dennoch verbleibt bei dem derartigen Kugelgelenk eine gewisse Restelastizität in weiteren Freiheitsgraden, insbesondere wenn das Kugelgelenk innen und/oder außen mit einem Dämpfungskörper gekoppelt ist.

Der Drehstabstabilisator ist vorteilhaft mit einem Längenausgleichselement versehen, welches es gestattet, dass sich ein Endbereich des Drehstabstabilisators in Querrichtung des zugehörigen Fahrzeugs bewegt, ohne dass es Auswirkungen auf das gegenüberliegende Ende des Drehstabstabilisators und das zugehörige Rad hätte. Das Längenausgleichelement ist dazu eingerichtet, das am Drehstabstabilisator an einer Radseite wirkende Drehmoment auf die andere Radseite hin zu übertragen. Dabei ist das Längenausgleichselement besonders bevorzugt als ein sog. Ball-Spline gestaltet. Ein solches Ball-Spline in Form einer verdrehgesicherten Kugelführung mit Längsfreiheitsgrad schafft eine reibungsarme Drehmomentenübertragung mittels Kugeln, die in zugehörigen Nuten an einem Nabenabschnitt des Längenausgleichselements sowie einer diesen umfassenden Hülse angeordnet sind.

Der derartige erfindungsgemäße Drehstabstabilisator ist vorzugsweise nur an seinen beiden Endbereichen dazu angepasst an einer zugehörigen Fahrzeugkarosserie gelagert zu werden..

Nachfolgend wird ein Ausführungsbeispiel der erfindungsgemäßen Lösung anhand der beigefügten schematischen Zeichnungen näher erläutert. Es zeigt:
- Fig. 1: eine perspektivische Ansicht einer Achse gemäß dem Stand der Technik,
- Fig. 2: die Ansicht II gemäß Fig. 1,
- Fig. 3: die Ansicht III gemäß Fig. 1,
- Fig. 4: eine perspektivische Ansicht eines Längslenkers und eines Drehstabstabilisators der Achse gemäß Fig. 1,
- Fig. 5: eine perspektivische Ansicht eines ersten Ausführungsbeispiels einer erfindungsgemäßen Achse,
- Fig. 6: eine perspektivische Ansicht eines Längslenkers und eines Drehstabstabilisators der Achse gemäß Fig. 5,
- Fig. 7: eine Detailansicht der Verbindung von Längslenker und Drehstabstabilisator gemäß Fig. 6,
- Fig. 8: die Ansicht VIII gemäß Fig. 7,
- Fig. 9: eine perspektivische Ansicht eines zweiten Ausführungsbeispiels einer erfindungsgemäßen Achse,
- Fig. 10: eine perspektivische Ansicht eines Längslenkers und eines Drehstabstabilisators der Achse gemäß Fig. 9,
- Fig. 11: eine Detailansicht der Verbindung von Längslenker und Drehstabstabilisator gemäß Fig. 10,
- Fig. 12: die Ansicht XII gemäß Fig. 11,
- Fig. 13: die Ansicht XIII gemäß Fig. 11 und
- Fig. 14: die Ansicht XIV gemäß Fig. 11.

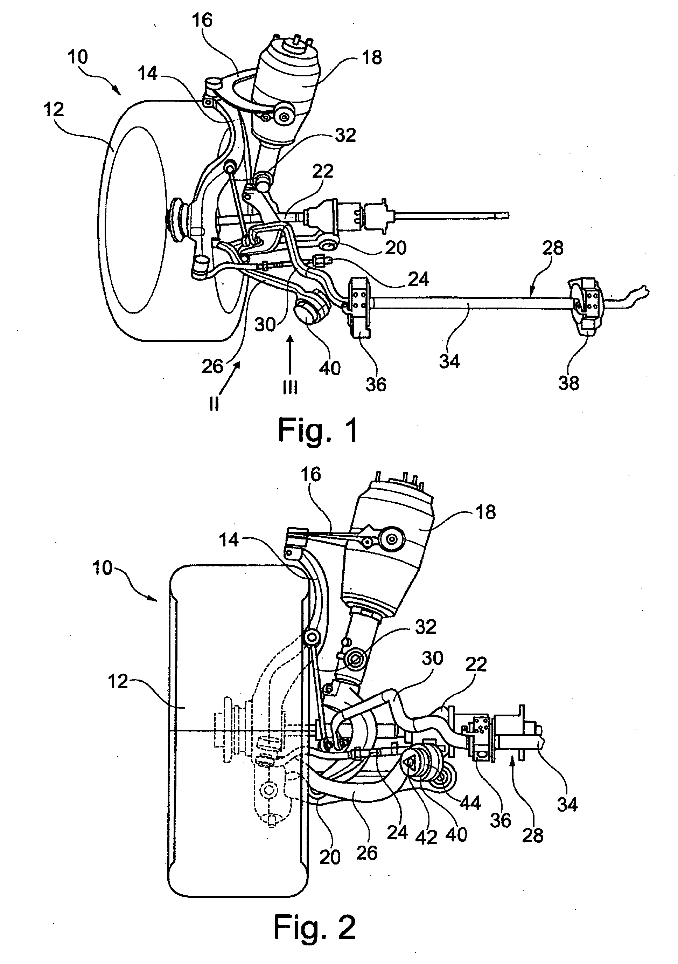

In den Fig. 1 bis 4 ist eine als antreibbare Vorderachse eines Kraftfahrzeugs gestaltete Achse 10 gemäß dem Stand der Technik veranschaulicht, bei der ein Rad 12 an einem Radträger 14 drehbar gelagert ist. Der Radträger 14 ist mit einem doppelarmigen oberen Querlenker 16 sowie einem Federbein 18 und mit einem unteren Querlenker 20 an einer weiter nicht veranschaulichten Fahrzeugkarosserie eines zugehörigen Fahrzeugs angelenkt. Eine Spurstange 24 bestimmt den Spurwinkel des Radträgers 14 bzw. Rades 12 und es führt eine Antriebswelle 22 zu dem am Radträger 14 drehbaren Rad 12.

Im unteren Bereich des Radträgers 14, der so genannten unteren Lenkerebene ist neben dem unteren Querlenker 20 und der Spurstange 24 an der Achse 10 ein schräg zur Hauptfahrtrichtung ausgerichteter Längslenker 26 angeordnet. Der Längslenker 26 bildet insbesondere eine Zugstrebe zum Aufnehmen der während der Fahrt am Rad wirkenden Zugkräfte.

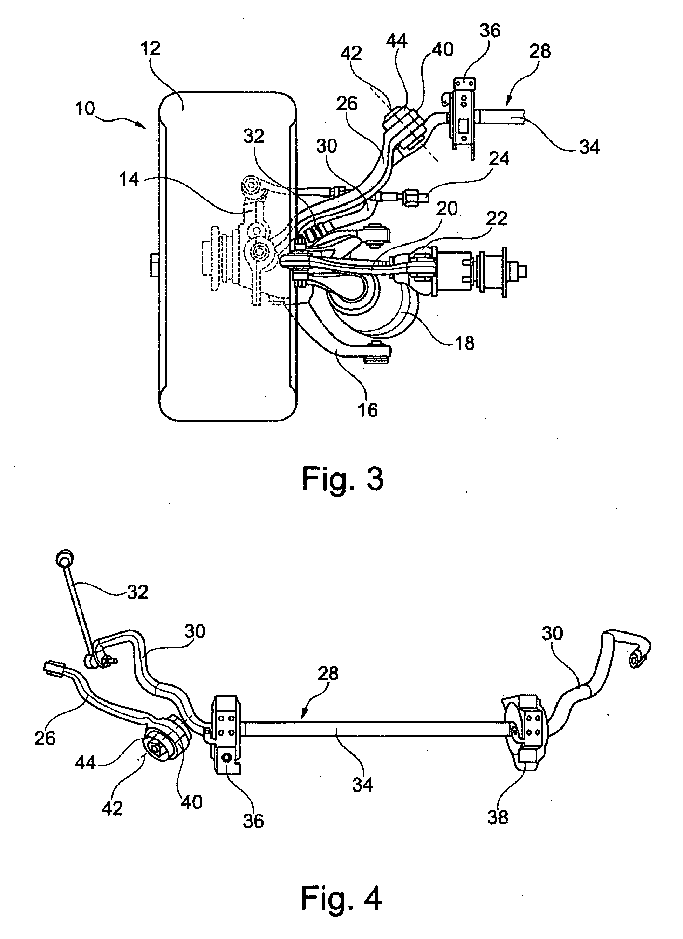

Ferner ist an der Achse 10 ein stabförmiger, aus Federstahl hergestellter Drehstabstabilisator 28 vorgesehen, den an seinen beiden Seiten je einen abgebogenen Endabschnitt 30 aufweist. Diese Endabschnitte 30 sind jeweils mittels einer Pendelstütze 32 am Radträger 14 angelenkt. Ein als Querstab 34 ausgebildeter Mittelbereich des Drehstabstabilisators 28 ist mittels einer ersten Abstützung 36 und einer zweiten Abstützung 38 ebenfalls an der Fahrzeugkarosserie in Querrichtung des Fahrzeugs schwenkbar abgestützt. Dies ist insbesondere in Fig. 4 dargestellt. Der Drehstabstabilisator 28 verbindet auf diese Weise die beiden Spurseiten der Achse 10 miteinander und reduziert damit die Wankneigung des zugehörigen Fahrzeugs insbesondere bei Kurvenfahrt.

Der Längslenker 26 ist bei dieser Achse 10 gemäß dem Stand der Technik in klassischer Weise an der Fahrzeugkarosserie mittels eines Schwenkgelenks 40 angelenkt und um eine im Wesentlichen sich horizontal und schräg zur Fahrzeuglängsrichtung erstreckende Schwenkachse 42 schwenkbar. Die Schwenkachse 42 ist dabei von einem Dämpfungskörper 44 in Gestalt einer Elastomerhülse umgeben.

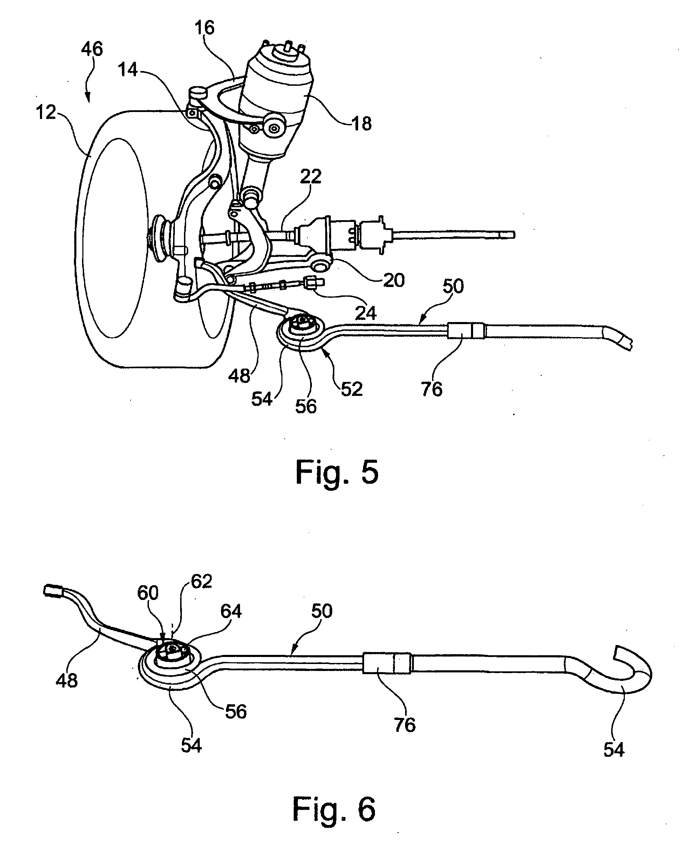

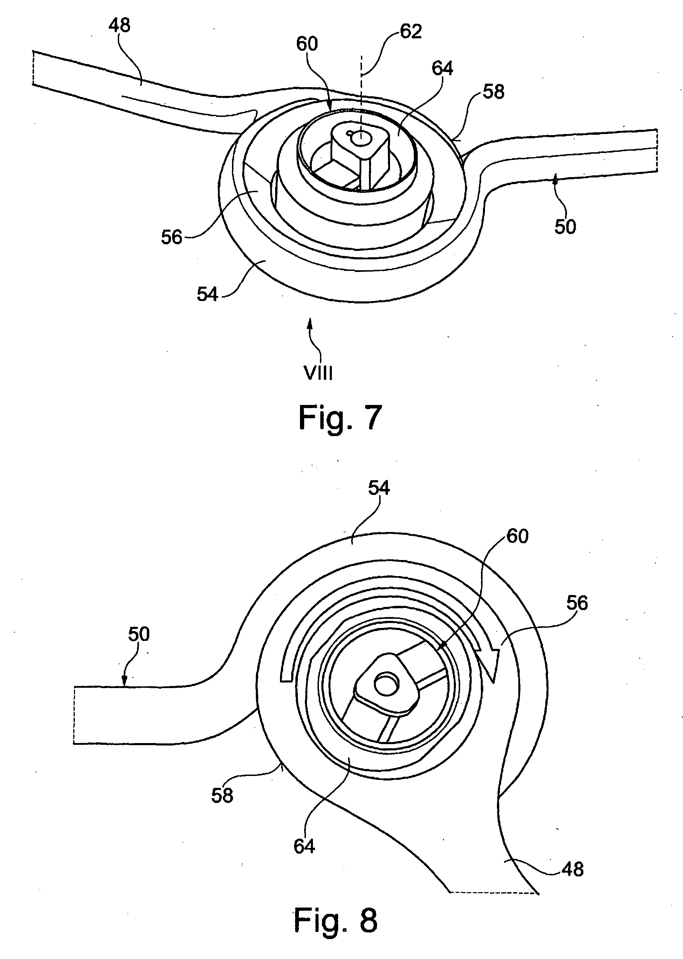

In den Fig. 5 bis 8 ist ein erstes Ausführungsbeispiel einer Achse 46 für lenkbare Räder 12 gemäß der Erfindung dargestellt. Die Achse 46 entspricht hinsichtlich der Komponenten 12, 14, 16, 18, 20, 22 und 24 im Wesentlichen der Achse 10 gemäß dem Stand der Technik.

Im Gegensatz zu der bekannten Achse 10 ist bei der Achse 46 hingegen eine Verbindung zwischen einem dortigen Längslenker 48 und einem dortigen Drehstabstabilisator 50 vorgesehen, womit keine Pendelstütze benötigt wird und deren Bauraumbedarf entfällt. Die Verbindung ist mittels eines Schwenkgelenks 52 gestaltet, bei dem ein im Wesentlichen halbkreisförmiger Endbereich 54 des Drehstabstabilisators 50 einen im Wesentlichen kreisringscheibenförmigen Endbereich 56 des Längslenkers 48 umgreift. Mit der derartigen Verbindung ist eine hinsichtlich einer Torsion des Drehstabilisators 50 drehmomentenübertragende Kopplung zwischen dem Drehstabstabilisator 50 und dem Längslenker 48 hergestellt, die dennoch eine gewisse Relativbewegung zwischen den Bauteilen 48 und 50 ermöglicht.

Für die zumindest geringfügige Bewegungsfreiheit der Bauteile 48 und 50 relativ zueinander ist an dem kreisringscheibenförmigen Endbereich 56 des Längslenkers 48 eine Gleitfläche 58 in Form eines inneren Teils eines Torus vorgesehen. An dieser Gleitfläche 58 gleitet der halbkreisförmig gebogene Endbereich 54 des Drehstabstabilisators 50 formschlüssig entlang.

Im Inneren des ringscheibenförmigen Endbereichs 56 des Längslenkers 48 ist ein Gelenk 60 zum Abstützen des Längslenkers 48 an einer nicht dargestellten Fahrzeugkarosserie vorgesehen. Das Gelenk 60 ist mit einer drehfest an der Fahrzeugkarosserie festzulegenden, sich im Wesentlichen in Richtung der Hochachse des zugehörigen Fahrzeugs erstreckende Achse 62 gestaltet. Die Achse 62 ist von einem Dämpfungskörper 64 in Gestalt einer hohlzylindrischen Elastomerhülse umgeben. Diese Elastomerhülse ist an ihrer Mantelfläche von dem Endbereich 56 des Längslenkers 48 umfasst.

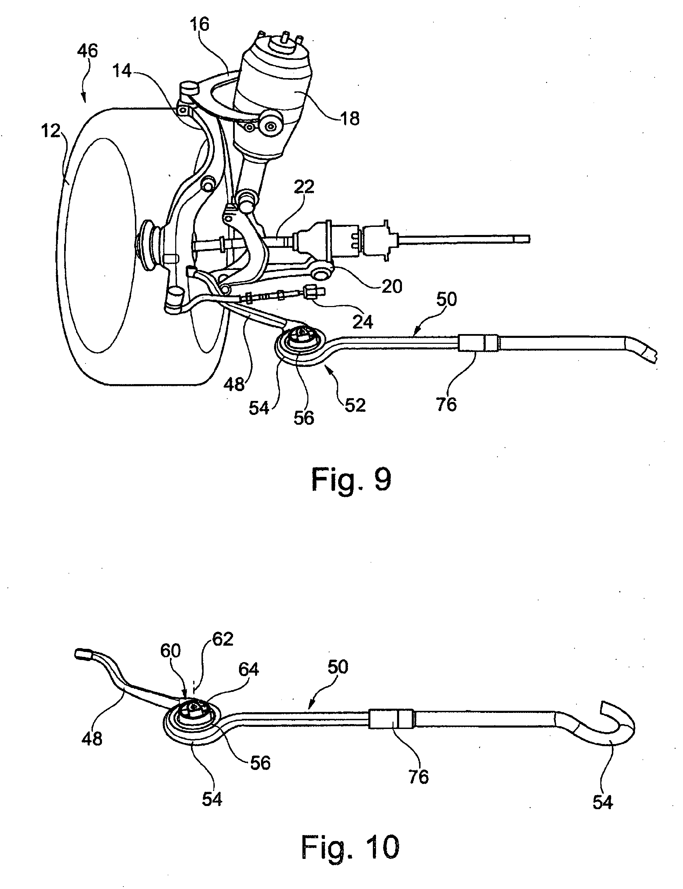

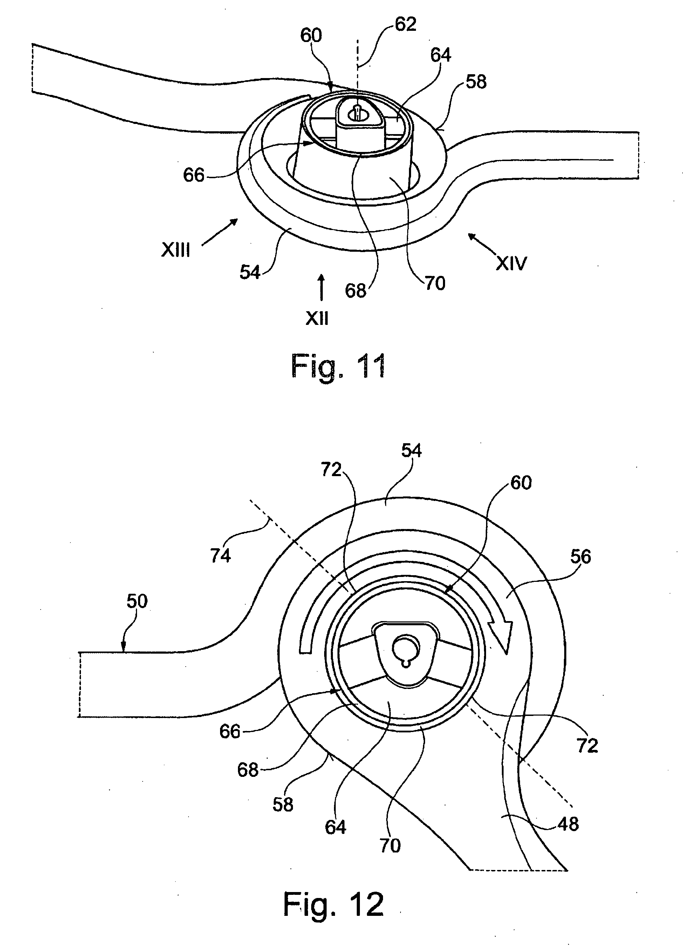

In den Fig. 9 bis 14 ist ein zweites Ausführungsbeispiel einer Achse 46 für lenkbare Räder 12 gemäß der Erfindung dargestellt. Die Achse 46 entspricht ebenfalls hinsichtlich der Komponenten 12, 14, 16, 18, 20, 22 und 24 im Wesentlichen der Achse 10 gemäß dem Stand der Technik. Im Übrigen ist die Achse 46 gemäß diesen Fig. ebenfalls mit einer Verbindung zwischen dem dortigen Längslenker 48 und dem dortigen Drehstabstabilisator 50 gestaltet.

Die Verbindung weist wiederum ein Schwenkgelenk 52 auf, bei dem ein im Wesentlichen halbkreisförmiger Endbereich 54 des Drehstabstabilisators 50 einen im Wesentlichen kreisringscheibenförmigen Endbereich 56 des Längslenkers 48 ähnlich dem zuvor erläuterten Ausführungsbeispiel umgreift.

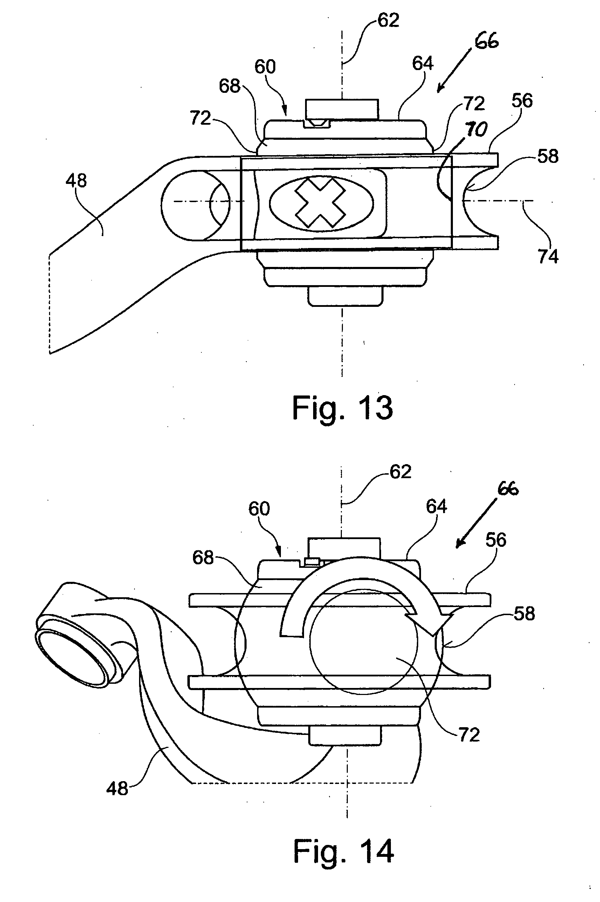

Im Zentrum des kreisringscheibenförmigen Endbereichs 56 des Längslenkers 48 ist ein Kugelgelenk 66 zum Abstützen des Längslenkers 48 an einer nicht dargestellten Fahrzeugkarosserie vorgesehen. Das Kugelgelenk 66 ist im Zentrum mit einem hohlzylindrischen Dämpfungskörper 64 und einer diesen umgebenden Kugel 68 gestaltet, die an ihrem Äquator von einer Kugelschale 70 umgriffen ist. Die Kugel 68 und die zugehörige Kugelschale 70 weisen dabei an zwei gegenüberliegenden Seite je eine Abflachung 72 auf, mittels denen eine Schwenkachse 74 für den Längslenker 48 an dem Kugelgelenk 66 gestaltet ist, indem die aufeinander aufliegenden Abflachungen 72 der Kugel 68 und der Kugelschale 72 gemäß dem in Fig.14 dargestellten Pfeil um die in Fig.14 senkrecht zur Zeichenebene verlaufende Schwenkachse 74 aufeinander gleiten.

An den Drehstabstabilisatoren 50 gemäß den Fig. 5 bis 14 ist ferner je etwa mittig von deren Längserstreckung ein Längenausgleichselement 76 in Gestalt eines Ball-Splines (=verdrehgesicherten Kugelführung mit Längsfreiheitsgrad) vorgesehen. Das Längenausgleichselement 76 ermöglicht einen Längenausgleich zwischen den beiden Schwenkgelenken 52 an den beiden Enden des Drehstabilisators 50, insbesondere wenn sich an den Dämpfungskörpern 64 Verformungen ergeben oder ein Schwenken an den Kugelgelenken 66 stattfindet. Zugleich ist das Längenausgleichselement 76 so gestaltet, dass es am Drehstabstabilisator 50 an einer Radseite wirkende Drehmomente auf die andere Radseite hin überträgt.

## Patentansprüche

1. Achse (46) für Räder eines zweispurigen Fahrzeugs, mit einer einen Radträger (14) führenden, aufgelösten unteren Lenkerebene, die durch einen Querlenker (20) sowie einen Längslenker (48) gebildet ist, sowie mit einem Drehstabstabilisator (50), der um eine im wesentlichen in Fahrzeug-Querrichtung verlaufende Drehachse verdrehbar und mit seinem Endbereich über eine drehmomentenübertragende Verbindung mit dem vom Radträger (14) abgewandten Endbereich des Längslenkers (48) verbunden ist, wobei diese Verbindung mit einem Schwenkgelenk (52) gebildet ist, bei dem der Endbereich (54) des Drehstabstabilisators (50) den Endbereich (56) des Längslenkers (48) schwenkbar umgreift,
**dadurch gekennzeichnet, dass** der Endbereich (54) des Drehstabstabilisators (50) den Endbereich (56) des Längslenkers (48) im Wesentlichen zumindest halbkreisförmig umgreift.

2. Achse nach Anspruch 1,
**dadurch gekennzeichnet, dass** das Schwenkgelenk (52) als ein Gleitlager des Drehstabstabilisators (50) am Längslenker (48) gestaltet ist.

3. Achse nach Anspruch 1 oder 2,
**dadurch gekennzeichnet, dass** der Längslenker (48) mit einem Dämpfungskörper (64) zum federnden Abstützen des Längslenkers (48) an einer zugehörigen Fahrzeugkarosserie gestaltet ist und der Dämpfungskörper (64) im Endbereich des Längslenkers (48) angeordnet ist.

4. Achse nach Anspruch 3,
**dadurch gekennzeichnet, dass** der Dämpfungskörper (64) im Zentrum des Schwenkgelenks (52) angeordnet ist.

5. Achse nach einem der Ansprüche 1 bis 4,
**dadurch gekennzeichnet, dass** der Längslenker (48) mit einem Gelenk zum Abstützen des Längslenkers (48) an einer zugehörigen Fahrzeugkarosserie gestaltet ist und das Gelenk als Kugelgelenk (66) gestaltet ist.

6. Achse nach Anspruch 5,
**dadurch gekennzeichnet, dass** das Kugelgelenk (66) im Zentrum des Schwenkgelenks (52) angeordnet ist.

7. Achse nach Anspruch 5 oder 6,
**dadurch gekennzeichnet, dass** das Kugelgelenk (66) mit mindestes einer seitlichen Abflachung zum Festlegen einer definierten Rotationsachse des Längslenkers (48) an der Fahrzeugkarosserie versehen ist.

8. Achse nach einem der vorfangegangenen Ansprüche,
**dadurch gekennzeichnet, dass** der Drehstabstabilisator (50) mit einem Längenausgleichselement versehen ist.

9. Achse nach einem der Ansprüche 1 bis 8,
**dadurch gekennzeichnet, dass** der Drehstabstabilisator (50) nur an seinen beiden Endbereichen (54) dazu angepasst ist an einer zugehörigen Fahrzeugkarosserie gelagert zu werden.

## Claims

1. An axle (46) for wheels of a two-track vehicle, with a theoretical lower control arm plane, which guides a wheel carrier (14) and is formed by a transverse control arm (20) and a longitudinal control arm (48), and with a torsion bar stabiliser (50), which can be rotated about a rotational axis running substantially in the transverse direction of the vehicle and is connected by its end region by means of a torque-transmitting connection to the end region of the longitudinal control arm (48) remote from the wheel carrier (14), wherein this connection is formed by a pivot joint (52), in which the end region (54) of the torsion bar stabiliser (50) pivotably encompasses the end region (56) of the longitudinal control arm (48), **characterised in that** the end region (54) of the torsion bar stabiliser (50) substantially encompasses the end region (56) of the longitudinal control arm (48) at least in a semicircle.

2. An axle according to claim 1, **characterised in that** the pivot joint (52) is configured as a sliding bearing of the torsion bar stabiliser (50) on the longitudinal control arm (48).

3. An axle according to claim 1 or 2, **characterised in that** the longitudinal control arm (48) is configured with a damping body (64) for the resilient support of the longitudinal control arm (48) on an associated vehicle body and the damping body (64) is arranged in the end region of the longitudinal control arm (48).

4. An axle according to claim 3, **characterised in that** the damping body (64) is arranged in the centre of the pivot joint (52).

5. An axle according to any one of claims 1 to 4, **characterised in that** the longitudinal control arm (48) is configured with a joint for supporting the longitudinal control arm (48) on an associated vehicle body and the joint is configured as a ball joint (66).

6. An axle according to claim 5, **characterised in that** the ball joint (66) is arranged in the centre of the pivot joint (52).

7. An axle according to claim 5 or 6, **characterised in that** the ball joint (66) is provided with at least one lateral flattened area for fixing a defined rotational axis of the longitudinal control arm (48) on the vehicle body.

8. An axle according to any one of the preceding claims, **characterised in that** the torsion bar stabiliser (50) is provided with a longitudinal compensation element.

9. An axle according to any one of claims 1 to 8, **characterised in that** the torsion bar stabiliser (50) is only adapted at its two end regions (54) to be mounted on an associated vehicle body.

## Revendications

1. Essieu (46) de roues d'un véhicule à deux voies comprenant un plan inférieur de bras divisé, guidant un support de roues (14), ce plan étant défini par un bras transversal (20) et un bras longitudinal (48),
- ainsi qu'un stabilisateur de barre d'accouplement (50) qui peut tourner autour d'un axe de rotation passant essentiellement dans la direction transversale du véhicule et relié par sa zone d'extrémité, par une liaison de transmission de couple à la zone d'extrémité du bras longitudinal (48) non tourné vers le support de roue (14),
- cette liaison étant formée d'une articulation de pivotement (52) dans laquelle la zone d'extrémité (54) du stabilisateur de barre d'accouplement (50) entoure de façon pivotante la zone d'extrémité (56) du bras longitudinal (48),
essieu **caractérisé en ce que**
la zone d'extrémité (54) du stabilisateur de barre d'accouplement (50) entoure la zone d'extrémité (56) du bras longitudinal (48) pratiquement au moins suivant une forme de demi-cercle.

2. Essieu selon la revendication 1,
**caractérisé en ce que**
l'articulation de pivotement (52) est réalisée sous la forme d'un palier lisse du stabilisateur de barre d'accouplement (50) sur le bras longitudinal (48).

3. Essieu selon la revendication 1 ou 2,
**caractérisé en ce que**
le bras longitudinal (48) est équipé d'un organe amortisseur (64) pour soutenir élastiquement le bras longitudinal (48) à la carrosserie du véhicule et l'organe amortisseur (64) est dans la zone d'extrémité du bras longitudinal (48).

4. Essieu selon la revendication 3,
**caractérisé en ce que**
l'organe d'amortissement (64) est au centre de l'articulation de pivotement (52).

5. Essieu selon la revendication 1 à 4,
**caractérisé en ce que**
le bras longitudinal (48) est muni d'une articulation pour soutenir le bras longitudinal (48) sur une partie correspondante de la carrosserie du véhicule et l'articulation est en forme d'articulation à rotule (66).

6. Essieu selon la revendication 5,
**caractérisé en ce que**
l'articulation à rotule (66) est au centre de l'articulation de pivotement (52).

7. Essieu selon la revendication 5 ou 6,
**caractérisé en ce que**
l'articulation à rotule (66) a au moins un aplatissement latéral pour fixer un axe de rotation défini du bras longitudinal (48) par rapport à la carrosserie du véhicule.

8. Essieu selon l'une des revendications précédentes,
**caractérisé en ce que**
le stabilisateur de barre d'accouplement (50) est muni d'un élément de compensation de longueur.

9. Essieu selon l'une des revendications 1 à 8,
**caractérisé en ce que**
le stabilisateur de barre d'accouplement (50) n'est adapté qu'à ces deux zones d'extrémité (54) pour être logé dans une partie correspondante de la carrosserie du véhicule.
